# EUROPÄISCHE PATENTSCHRIFT

(11) **EP 3 755 277 B1**
(45) Veröffentlichungstag und Bekanntmachung des Hinweises auf die Patenterteilung: **01.10.2025**
(21) Anmeldenummer: 19706545.1
(22) Anmeldetag: 18.02.2019
(51) Int. Cl.: A61F 2/90, D04C 1/06, A61F 2/06

(54) **GEFLOCHTENE STRUKTUR, INSBESONDERE STENT, UND VERFAHREN ZUM FLECHTEN EINER GEFLOCHTENEN STRUKTUR**
BRAIDED STRUCTURE, IN PARTICULAR STENT, AND METHOD FOR BRAIDING A BRAIDED STRUCTURE
STRUCTURE TRESSÉE, EN PARTICULIER STENT, ET PROCÉDÉ DE TRESSAGE D'UNE STRUCTURE TRESSÉE

(30) Priorität: 19.02.2018 DE 102018103613
(43) Veröffentlichungstag der Anmeldung: 30.12.2020
(73) Patentinhaber: Freistaat Bayern vertreten durch Hochschule Hof, Institut für Materialwissenschaften, 95028 Hof/Saale (DE)
(72) Erfinder: FICKER, Frank, 08626 Tiefenbrunn (DE); MIKSCH, Roxana, 63695 Glauberg (DE)
(74) Vertreter: Canzler & Bergmeier Patentanwälte Partnerschaft mbB
(86) Internationale Anmeldenummer: PCT/EP2019/053991
(87) Internationale Veröffentlichungsnummer: WO 2019/158760

(56) Entgegenhaltungen:
- EP-A1- 2 008 623
- EP-A2- 1 148 839
- WO-A1-2015/071336
- US-A1- 2002 007 210
- KUEPPERS ET AL: "Flechten von Verzweigungen fuer die Faserverbundtechnik", MELLIAND TEXTILBERICHTE, DEUTSCHER FACHVERLAG, FRANKFURT AM MAIN, DE, vol. 2, no. 2, 1 January 2016 (2016-01-01), pages 76 - 77, XP009512894, ISSN: 0341-0781

## Beschreibung

Die vorliegende Erfindung betrifft eine geflochtene Struktur, insbesondere einen Stent, mit einem primären Schenkel und mindestens zwei sekundären Schenkeln, wobei der primäre Schenkel aus einem Ensemble an Filamenten, insbesondere Fäden oder Drähten, geflochten ist und die sekundären Schenkel aus Filamenten geflochten sind, so dass die Gesamtheit der den sekundären Schenkeln zugeordneten Filamente gleich dem Ensemble an Filamenten des primären Schenkels ist. Des Weiteren betrifft die Erfindung ein Verfahren zum Flechten einer geflochtenen Struktur, insbesondere eines Stents, mit einem primären Schenkel und mindestens zwei sekundären Schenkeln, wobei aus einem Ensemble an Filamenten, insbesondere Fäden oder Drähten, zunächst der primäre Schenkel und dann die sekundären Schenkel geflochten werden, oder die Verfahrensschritte in umgekehrter Reihenfolge durchgeführt werden.

Bei gattungsgemäßen geflochtenen Strukturen wird beispielsweise zunächst ein primärer Schenkel aus einem Ensemble an Filamenten geflochten. Die Klöppel für einen ersten sekundären Schenkel werden sodann in der Flechtmaschine neu angeordnet, während die Klöppel für einen oder mehrere weitere sekundäre Schenkel geparkt werden. Anschließend, wenn der erste sekundäre Schenkel geflochten ist, werden nacheinander die weiteren sekundären Schenkel geflochten, während die Klöppel für die jeweils anderen sekundären Schenkel geparkt werden. So wird weiter verfahren, bis alle sekundären Schenkel geflochten sind.

Durch das Parken und das notwendige manuelle Umsortieren der Klöppel gestaltet sich dieses Verfahren als sehr aufwendig. Außerdem entstehen zwischen den sekundären Schenkeln Löcher, die einer optimalen Funktion der geflochtenen Struktur als Stent entgegenstehen.

Die einzelnen Schenkel gemäß der Offenbarung des Artikels KUEPPERS ET AL: "Flechten von Verzweigungen für die Faserverbundtechnik", MELLIAND TEXTILBERICHTE, DEUTSCHER FACHVERLAG, FRANKFURT AM MAIN, DE, Bd. 2, Nr. 2, 1. Januar 2016 (2016-01-01), Seiten 76-77, XP009512894, ISSN: 0341-0781 werden aus Filamenten geflochten, welche aus zwei benachbarten Schenkeln stammen. Dort heißt es, dass jeder Ast mit seinen beiden Nachbarn verbunden ist. Nicht alle Fäden eines Astes kommen aus dem Stamm oder einem anderen Ast. Jeder Ast teilt sich die Fäden von seinen beiden Nachbarn. Nach der Verzweigung werden zusätzliche Fäden hinzugefügt. Jeder Ast, egal ob primär oder sekundär, weist somit immer dieselbe Fadenanzahl auf.

Aus der EP 1148839 A2 sind Stents bekannt, die einen ersten Schenkel, der aus einer ersten Vielzahl von Drähten gebildet wird, einen zweiten Schenkel, der aus einer zweiten Vielzahl von Drähten gebildet wird, und einen gemeinsamen Körper, der aus der ersten und zweiten Vielzahl von Drähten gebildet wird, umfassen. Die Drähte können aus Nitinol bestehen, es können aber auch biologisch abbaubare Fäden verwendet werden. Die zwischen den gekreuzten Drähten gebildeten Winkel sind vorzugsweise stumpfwinklig. Sie können durch einfaches Flechten, entweder von Hand oder maschinell, gebildet werden. Die einzelnen Schenkel werden nach ihrer Herstellung miteinander verbunden.

Die US 2002/0007210 A1 offenbart einen Stent, der eine Vielzahl von miteinander verflochtenen Endlosfäden umfasst, wobei der Stent mindestens einen ersten Bereich mit einer ersten, relativ größeren Querschnittsfläche und mindestens einem zweiten Bereich mit einer zweiten, relativ kleineren Querschnittsfläche umfasst. In dem Übergangsbereich zwischen den beiden Querschnittsflächen entstehen meist Löcher in der Struktur.

Aufgabe der vorliegenden Erfindung ist es somit, eine verbesserte geflochtene Struktur und ein verbessertes Verfahren zum Flechten einer geflochtenen Struktur vorzuschlagen.

Die Aufgabe wird gelöst durch eine geflochtene Struktur und ein Verfahren zum Flechten einer geflochtenen Struktur mit den Merkmalen der unabhängigen Patentansprüche.

Vorgeschlagen wird eine geflochtene Struktur mit einem primären Schenkel und mindestens zwei sekundären Schenkeln. Eine solche geflochtene Struktur kann insbesondere ein Stent oder eine Struktur für eine Rohrleitung sein, wobei es sich hier um einen verzweigten Stent handelt. Stents finden dabei in der Medizin vielfältige Anwendung, beispielsweise zum Offenhalten von Blutgefäßen oder zum Offenhalten von Bronchien. Derartige Strukturen für Rohrleitungen können zur Verstärkung, Sanierung oder Reparatur von Rohrleitungen, die beispielsweise aus Kunststoff hergestellt sind, eingesetzt werden. Darüber hinaus sind vielfältige weitere Anwendungen derartiger Strukturen möglich.

Der primäre Schenkel ist dabei aus einem Ensemble an Filamenten geflochten. Unter Filament wird dabei ein quasi-endloses längliches Gebilde verstanden. Insbesondere kann es sich bei den Filamenten um Fäden oder Drähte handeln. Für medizinische Zwecke ist es oft vorteilhaft, wenn die Drähte aus einer Formgedächtnis-Legierung, wie beispielsweise Nitinol, bestehen.

Die sekundären Schenkel sind ebenfalls aus Filamenten geflochten, und zwar so, dass die Gesamtheit der den sekundären Schenkeln zugeordneten Filamente gleich dem Ensemble an Filamenten des primären Schenkels ist.

Anders ausgedrückt teilen sich die Filamente, aus denen der primäre Schenkel geflochten ist, in mehrere Teile auf, wobei aus jedem dieser Teil ein sekundärer Schenkel geflochten ist. Filamente aus sekundären Schenkeln werden nicht für die Herstellung anderer sekundärer Schenkel außerhalb des Übergangsbereichs benötigt.

Erfindungsgemäß ist zwischen dem primären Schenkel und den sekundären Schenkeln ein Übergangsbereich angeordnet, der aus dem Ensemble an Filamenten des primären Schenkels geflochten ist. Das Ensemble an Filamenten geht also nicht direkt vom primären Schenkel in die sekundären Schenkel über, sondern ist dazwischen zum Übergangsbereich geflochten. Ohne Übergangsbereich, also wenn der primäre Schenkel direkt in die sekundären Schenkel übergeht, tritt zwischen den sekundären Schenkeln ein Loch auf. Durch den entsprechend geflochtenen Übergangsbereich wird dieses Loch teilweise oder vollständig geschlossen. Es ergibt sich also eine, insbesondere für medizinische Zwecke, deutlich verbesserte geflochtene Struktur.

Diese verbesserte geflochtene Struktur weist in dem geflochtenen Übergangsbereich vorteilhafterweise im Wesentlichen kein Loch auf. Vorzugsweise ist weder in dem Zwickelbereich zwischen den sekundären Schenkeln, noch am äußeren Umfang des Übergangsbereichs ein Loch vorhanden. Dies bedeutet, dass die maximale Größe der einzelnen Maschen im Übergangsbereich im Wesentlichen der Größe der Maschen des primären bzw. sekundären Schenkels entspricht. Während eine kleinere Größe der Maschen im Übergangsbereich bei manchen Ausführungen vorteilhaft sein kann, sind größere Maschen, beispielsweise mit einer Fläche von mehr als dem 1,5-fachen der Maschenfläche in einem Schenkel, welche ein unzulässiges Loch darstellen, zu vermeiden.

Ein besonderer Vorteil der Erfindung ist es, dass die Herstellung der sekundären Schenkel gleichzeitig erfolgen kann. Die gesamte geflochtene Struktur wird damit durchgängig bzw. kontinuierlich und ohne erforderliche Pause für einen insbesondere manuellen Umbau der Flechtvorrichtung hergestellt. Ein manueller Eingriff, insbesondere bei der Herstellung des Übergangsbereichs, ist somit nicht erforderlich.

Die Herstellung der geflochtenen Struktur kann auch ohne Kern erfolgen. Das Einhängen unterschiedlicher Kerne für unterschiedliche Durchmesser der einzelnen Schenkel ist damit nicht erforderlich und fördert das kontinuierliche Flechten der Struktur. Durch das kontinuierliche Flechten der Struktur entstehen keine knotenartigen Verschlingungen der Filamente, sondern Verschlingungen in vertikaler Richtung, d. h. in Längsrichtung des Geflechts. Rein horizontale Verschlingungen oder rückwärts gerichtete Verschlingungen erfolgen nicht, da der Herstellungsfortschritt des Geflechts in vertikaler Richtung erfolgt.

Vorteilhafterweise ist die geflochtene Struktur durchgängig, insbesondere auf einem Flechter mit verstellbaren Weichen, geflochten. Ein solcher Flechter mit verstellbaren Weichen ist vorzugsweise ein Variationsflechter, ein Verzweigungsflechter oder ein 3D-Flechter. Dadurch, dass die Weichen verstellbar sind, ist es möglich, die geflochtene Struktur ohne beziehungsweise mit minimalem manuellem Eingriff zu flechten. Insbesondere können der Übergang vom primären Schenkel zum Übergangsbereich, der Übergangsbereich selbst und der Übergang vom Übergangsbereich zu den sekundären Schenkeln einfach durch Verstellen der Weichen geflochten werden. Ein Aus- und Einhängen, Umsortieren sowie Parken von Klöppeln ist dabei nicht notwendig, so dass die geflochtene Struktur relativ einfach herzustellen ist.

Von Vorteil ist es auch, wenn der Übergangsbereich einen Teilungsbereich umfasst, der eine Flechtstruktur des primären Schenkels in Flechtstrukturen der sekundären Schenkel überführt. Den einzelnen Flechtstrukturen entsprechen bestimmte Positionen der Klöppel in der Flechtmaschine, wobei in der Regel die Positionen der Klöppel beim Flechten des primären Schenkels nicht mit Positionen der Klöppel beim Flechten der sekundären Schenkel übereinstimmen. Um die Klöppel von der einen Position in die andere zu bringen, wird der Teilungsbereich geflochten. Dabei kann bei einer geschickten Weichenstellung schon eine Vierteldrehung von Flügelrädern der Flechtmaschine ausreichend sein.

Erfindungsgemäß umfasst der Übergangsbereich einen Überkreuzungsbereich, in dem sich Filamente, die verschiedenen sekundären Schenkeln zugeordnet sind, überkreuzen. Dabei kann der Überkreuzungsbereich auch gleichzeitig den Teilungsbereich umfassen. Durch das Überkreuzen der Filamente, die verschiedenen sekundären Schenkeln zugeordnet sind, wird das Loch zwischen diesen Schenkeln geschlossen. Das Überkreuzen kann dabei in vielfältiger Weise geschehen. Ein zunächst einem sekundären Schenkel zugeordnetes Filament kann nach dem Überkreuzen einem anderen sekundären Schenkel zugeordnet sein, und umgekehrt. Die Filamente können aber nach dem Überkreuzen auch wieder zu dem ihnen ursprünglich zugeordneten Schenkel zurückkehren. Des Weiteren ist es möglich, dass sich nicht nur jeweils ein Filament überkreuzt, sondern dass sich nebeneinander oder hintereinander mehrere Filamente überkreuzen.

Erfindungsgemäß überkreuzen sich im Überkreuzungsbereich auch Filamente, die von Seiten des sekundären Schenkels kommen, die dem jeweils anderen sekundären Schenkel abgewandt sind. Es überkreuzen sich also nicht nur Filamente von der Innenseite zwischen den Schenkeln, sondern auch von der Außenseite. Durch das Einbeziehen dieser Filamente wird das Loch zwischen den Schenkeln besonders gut geschlossen.

Von Vorteil ist es, wenn im Überkreuzungsbereich zwei Filamente, die insbesondere verschiedenen sekundären Schenkeln zugeordnet sind, untereinander verdreht sind. Ein untereinander Verdrehen um eine halbe Umdrehung entspricht dabei einem einfachen Überkreuzen, wobei die Filamente jeweils zu den ihnen ursprünglich zugeordneten Schenkeln zurückkehren. Zum besseren Verschließen des Lochs zwischen den Schenkeln ist aber ein untereinander Verdrehen von einem Vielfachen einer halben Umdrehung vorteilhaft. Bei einem ungeraden Vielfachen einer halben Umdrehung kehren die Filamente dabei jeweils zu den ihnen ursprünglich zugeordneten Schenkeln zurück, bei einem geraden Vielfachen einer halben Umdrehung wechseln sie den Schenkel.

Vorteilhaft ist es, wenn der Übergangsbereich einen weiteren Teilungsbereich umfasst, so dass der Überkreuzungsbereich zwischen dem Teilungsbereich und dem weiteren Teilungsbereich angeordnet ist. Im Teilungsbereich geht zunächst die Position der Klöppel von der Flechtstruktur für den primären Schenkel auf die Flechtstruktur für die sekundären Schenkel über. Im Überkreuzungsbereich werden sodann die Filamente der sekundären Schenkel so überkreuzt, dass die Löcher zwischen den sekundären Schenkeln geschlossen sind. Der weitere Teilungsbereich führt nun die Position der Klöppel, wie sie am Ende des Überkreuzungsbereichs ist, in eine Position über, die zum Flechten der sekundären Schenkel geeignet ist. So ist also ein durchgängiges Flechten der einzelnen Bereiche möglich, ohne dass Klöppel manuell verändert werden müssen.

In einer besonders vorteilhaften Ausführung der Erfindung sind an beiden Enden des primären Schenkels sekundäre Schenkel angeordnet. Damit können sehr komplexe geflochtene Strukturen erzeugt werden. Insbesondere ist es jedoch auch möglich, bei einem durchgängigen Flechten eine quasi endlose geflochtene Struktur zu erzeugen, welche eine Vielzahl aneinandergereihter primärer und sekundärer Schenkel aufweist, welche bei Bedarf in einzelne geflochtene Strukturen mit beispielsweise einem primären Schenkel und an nur einem Ende des primären Schenkels angeordneten sekundären Schenkeln getrennt werden kann.

Vorzugsweise ist an dem primären Schenkel und/oder dem sekundären Schenkel ein Trennbereich mit einer gegenüber dem restlichen Schenkel geänderter Maschendichte und/oder Maschenform zum Trennen der einen gesamten geflochtenen Struktur in mehrere einzelne geflochtene Strukturen vorgesehen. Der Trennbereich kann derart gestaltet sein, dass er ein Abtrennen, beispielsweise mit einem Laser, vorteilhafterweise ermöglicht und insbesondere die abgetrennten Filamente kompakt zusammenhält. Diese abgetrennten und gegebenenfalls abstehenden Filamente können zu Schlaufen eingeschlagen werden, um ein stumpfes Ende der geflochtenen Struktur zu bilden.

Des Weiteren wird ein Verfahren zum Flechten einer geflochtenen Struktur mit einem primären Schenkel und mindestens zwei sekundären Schenkeln vorgeschlagen. Die geflochtene Struktur kann dabei insbesondere ein Stent sein, wobei es sich hier um einen verzweigten Stent handelt.

Zunächst wird aus einem Ensemble an Filamenten der primäre Schenkel geflochten. Unter Filament wird dabei ein quasi-endloses längliches Gebilde verstanden. Insbesondere kann es sich bei den Filamenten um Fäden oder Drähte handeln, wobei es für medizinische Zwecke oft vorteilhaft ist, wenn die Drähte aus einer Formgedächtnis-Legierung wie beispielsweise Nitinol bestehen.

Erfindungsgemäß wird aus dem Ensemble an Filamenten des primären Schenkels sodann ein im Wesentlichen lochfreier Übergangsbereich geflochten. Ohne Übergangsbereich, also wenn der primäre Schenkel direkt in sekundäre Schenkel übergeht, tritt zwischen den sekundären Schenkeln ein Loch auf. Durch den entsprechend geflochtenen Übergangsbereich wird dieses Loch teilweise oder vollständig geschlossen. Das Verfahren ergibt also eine, insbesondere für medizinische Zwecke, deutlich verbesserte geflochtene Struktur.

Schließlich werden aus dem Ensemble an Filamenten des primären Schenkels die sekundären Schenkel geflochten. Das Ensemble an Filamenten wird also in mehrere Teile aufgeteilt, wobei aus jedem Teil ein sekundärer Schenkel geflochten wird.

Selbstverständlich können die Verfahrensschritte auch in umgekehrter Reihenfolge durchgeführt werden. Es können also zunächst die sekundären Schenkel, dann der Übergangsbereich und schließlich der primäre Schenkel geflochten werden.

Vorteilhafterweise wird die geflochtene Struktur durchgängig, insbesondere mit einem Flechter mit schaltbaren Weichen, vorzugsweise einem Variationsflechter, Verzweigungsflechter oder 3D-Flechter, geflochten. Dadurch, dass die Weichen verstellbar sind, ist es möglich, die geflochtene Struktur ohne beziehungsweise mit minimalem manuellem Eingriff zu flechten. Insbesondere können der Übergang vom primären Schenkel zum Übergangsbereich, der Übergangsbereich selbst und der Übergang vom Übergangsbereich zu den sekundären Schenkeln einfach durch Verstellen der Weichen geflochten werden. Ein Aus- und Einhängen, Umsortieren sowie Parken von Klöppeln ist dabei nicht notwendig, so dass die geflochtene Struktur relativ einfach herzustellen ist.

Von Vorteil ist es auch, wenn im Übergangsbereich die Filamente so geflochten werden, dass sie von einer Flechtstruktur des primären Schenkels in Flechtstrukturen der sekundären Schenkel überführt werden, wodurch ein Teilungsbereich ausgebildet wird. Den einzelnen Flechtstrukturen entsprechen bestimmte Positionen der Klöppel in der Flechtmaschine, wobei in der Regel die Positionen der Klöppel beim Flechten des primären Schenkels nicht mit Positionen der Klöppel beim Flechten der sekundären Schenkel übereinstimmen. Um die Klöppel von der einen Position in die andere zu bringen, wird der Teilungsbereich geflochten. Dabei kann bei einer geschickten Weichenstellung schon eine Vierteldrehung von Flügelrädern der Flechtmaschine ausreichend sein.

Erfindungsgemäß ist es, wenn im Übergangsbereich Filamente, die verschiedenen sekundären Schenkeln zugeordnet sind, überkreuzt werden und somit einen Überkreuzungsbereich ausbilden. Dabei kann der Überkreuzungsbereich auch gleichzeitig den Teilungsbereich umfassen. Durch das Überkreuzen der Filamente, die verschiedenen sekundären Schenkeln zugeordnet sind, wird das Loch zwischen diesen Schenkeln geschlossen. Das Überkreuzen kann dabei in vielfältiger Weise geschehen. Ein zunächst einem sekundären Schenkel zugeordnetes Filament kann nach dem Überkreuzen einem anderen sekundären Schenkel zugeordnet sein, und umgekehrt. Die Filamente können aber nach dem Überkreuzen auch wieder zu dem ihnen ursprünglich zugeordneten Schenkel zurückkehren. Des Weiteren ist es möglich, dass sich nicht nur jeweils ein Filament überkreuzt, sondern dass sich nebeneinander oder hintereinander mehrere Filamente überkreuzen.

Erfindungsgemäß werden im Überkreuzungsbereich auch Filamente überkreuzt, die von Seiten des sekundären Schenkels kommen, die dem jeweils anderen sekundären Schenkel abgewandt sind. Es werden also nicht nur Filamente von der Innenseite zwischen den Schenkeln überkreuzt, sondern auch von der Außenseite. Durch das Einbeziehen dieser Filamente wird das Loch zwischen den Schenkeln besonders gut geschlossen.

Von Vorteil ist es, wenn im Überkreuzungsbereich zwei Filamente, die insbesondere verschiedenen sekundären Schenkeln zugeordnet sind, untereinander verdreht werden. Ein untereinander Verdrehen um eine halbe Umdrehung entspricht dabei einem einfachen Überkreuzen, wobei die Filamente jeweils zu den ihnen ursprünglich zugeordneten Schenkeln zurückkehren. Zum besseren Verschließen des Lochs zwischen den Schenkeln ist aber ein untereinander Verdrehen von einem Vielfachen einer halben Umdrehung vorteilhaft. Bei einem ungeraden Vielfachen einer halben Umdrehung kehren die Filamente dabei jeweils zu den ihnen ursprünglich zugeordneten Schenkeln zurück, bei einem geraden Vielfachen einer halben Umdrehung wechseln sie den Schenkel.

Werden im Überkreuzungsbereich auch Filamente miteinander verdreht, die von Seiten des sekundären Schenkels kommen, die dem jeweils anderen sekundären Schenkel abgewandt sind, so können die Löcher sehr dicht geschlossen werden, da mehr Filamente zur Verfügung stehen, welche im Bereich des möglichen Loches geflochten werden.

Vorteilhaft ist es, wenn im Übergangsbereich ein weiterer Teilungsbereich geflochten wird, so dass zunächst der Teilungsbereich, dann der Überkreuzungsbereich und schließlich der weitere Teilungsbereich geflochten werden. Im Teilungsbereich geht zunächst die Position der Klöppel von der Flechtstruktur für den primären Schenkel auf die Flechtstruktur für die sekundären Schenkel über. Im Überkreuzungsbereich werden sodann die Filamente der sekundären Schenkel so überkreuzt, dass die Löcher zwischen den sekundären Schenkeln geschlossen sind. Der weitere Teilungsbereich führt nun die Position der Klöppel, wie sie am Ende des Überkreuzungsbereichs ist, in eine Position über, die zum Flechten der sekundären Schenkel geeignet ist. So ist also ein durchgängiges Flechten der einzelnen Bereiche möglich, ohne dass Klöppel manuell verändert werden müssen.

Vorzugsweise wird eine geflochtene Struktur, insbesondere in einem Trennbereich, in mehrere geflochtene Strukturen getrennt. Hierdurch kann in einem durchgängigen, kontinuierlichen Herstellungsverfahren ein Strang einer geflochtenen Struktur erzeugt werden, bei welchem sich primäre und sekundäre Schenkel abwechseln. An beliebigen oder auch an durch die Trennbereiche vorgegebenen Stellen kann dieser Strang in einzelne, kleinere geflochtene Strukturen aufgetrennt werden.

Die geflochtene Struktur ist gemäß der vorangegangenen Beschreibung ausgebildet und das Verfahren zum Flechten einer geflochtenen Struktur wird gemäß der vorangegangenen Beschreibung durchgeführt, wobei die genannten Merkmale einzeln oder in beliebiger Kombination vorhanden sein können.

Weitere Vorteile der Erfindung sind in den nachfolgenden Ausführungsbeispielen beschrieben. Es zeigt:
- **Figur 1**: eine schematische Ansicht einer aus dem Stand der Technik bekannten geflochtenen Struktur,
- **Figur 2**: eine schematische Ansicht einer weiteren geflochtenen Struktur,
- **Figur 3**: eine schematische Ansicht einer weiteren geflochtenen Struktur,
- **Figur 4**: eine schematische Ansicht einer weiteren geflochtenen Struktur,
- **Figur 5**: eine schematische Ansicht eines Überkreuzungsbereichs,
- **Figur 6**: eine schematische Ansicht eines weiteren Überkreuzungsbereichs,
- **Figur 7**: eine schematische Ansicht eines weiteren Überkreuzungsbereichs,
- **Figur 8**: eine schematische Ansicht eines weiteren Überkreuzungsbereichs,
- **Figur 9**: eine schematische Ansicht eines weiteren Überkreuzungsbereichs,
- **Figuren 10a -10c**: Flechtprogrammschritte für einen Teilungsbereich,
- **Figuren 11a** - **11e**: Flechtprogrammschritte für einen Übergangsbereich und
- **Figur 12**: eine schematische Ansicht einer weiteren geflochtenen Struktur ähnlich Figur 2 mit einem Trennbereich.

Bei der nachfolgenden Beschreibung alternativer Ausführungsbeispiele werden für Merkmale, die im Vergleich zu anderen Ausführungsbeispielen in ihrer Ausgestaltung und/oder Wirkweise identisch und/oder zumindest vergleichbar sind, gleiche Bezugszeichen verwendet. Sofern diese nicht nochmals detailliert erläutert werden, entspricht deren Ausgestaltung und/oder Wirkweise der Ausgestaltung und Wirkweise der vorstehend bereits beschriebenen Merkmale.

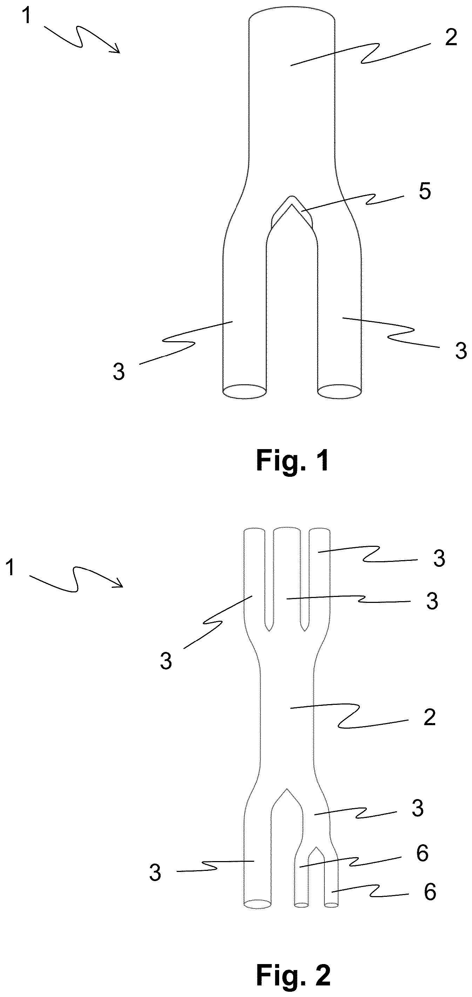

In Figur 1 ist eine geflochtene Struktur 1 gezeigt, die aus dem Stand der Technik bekannt ist. Die geflochtene Struktur 1 weist einen primären Schenkel 2 und zwei sekundäre Schenkel 3 auf. Die einzelnen Filamente 4, aus denen die geflochtene Struktur 1 geflochten ist, sind in dieser Figur nicht dargestellt. Dabei ist die Gesamtheit der Filamente 4, aus denen die sekundären Schenkel 3 geflochten sind, gleich dem Ensemble an Filamenten 4, aus denen der primäre Schenkel 2 geflochten ist. Als Filamente 4 kommen dabei unterschiedlichste quasi-endlose längliche Gebilde in Betracht, insbesondere Fäden und Drähte.

Beim Übergang vom primären Schenkel 2 auf die sekundären Schenkel 3 entsteht zwischen den sekundären Schenkeln 3 ein Loch 5. Dieses Loch 5 ist für viele Anwendungsmöglichkeiten der geflochtenen Struktur 1 von Nachteil. Eine solche Anwendungsmöglichkeit ist die Verwendung der geflochtenen Struktur 1 als Stent. Der Stent hält dabei Gefäße oder Hohlorgane offen, insbesondere Blutgefäße oder Bronchien. Bei der Verwendung der geflochtenen Struktur 1 als Stent bestehen die Filamente 4, vorteilhafterweise Drähte, aus einer Formgedächtnis-Legierung wie beispielsweise Nitinol. So kann der Stent zum Beispiel zusammengebunden werden, um in das Gefäß oder Hohlorgan eingeführt zu werden und entfaltet sich nach Lösen der Bindung zu seiner ursprünglichen Form. Ein Loch 5 zwischen den sekundären Schenkeln 3 eines verzweigten Stents bedeutet, dass in diesem Bereich weder eine Stützung der Gefäße beziehungsweise Hohlorgane auftritt, noch in diesem Bereich wirksam Medikamente auf die Oberfläche des Stents aufgebracht werden können.

Figur 2 zeigt eine schematische Ansicht einer erfindungsgemäßen geflochtenen Struktur 1, bei der die Löcher 5 zwischen den sekundären Schenkeln 3 geschlossen sind. Bei dieser komplexen geflochtenen Struktur 1 verzweigt sich der primäre Schenkel 2 in die eine Richtung in drei sekundäre Schenkel 3. In die andere Richtung verzweigt sich der primäre Schenkel 2 zunächst in zwei sekundäre Schenkel 3, wobei sich einer der sekundären Schenkel 3 in zwei weitere Schenkel 6 verzweigt. Im Hinblick auf die Verzweigung und die im Rahmen dieser Verzweigung auftretenden Flechtmuster fungiert der sekundäre Schenkel 3 hier als primärer Schenkel und die weiteren Schenkel 6 als sekundäre Schenkel. Selbstverständlich sind im Rahmen der Patentansprüche viele weitere Verzweigungsvarianten möglich, von einer einfachen verzweigten Struktur, wie sie in Figur 1 gezeigt ist, bis zu komplexen Strukturen mit einer Vielzahl an Verzweigungen.

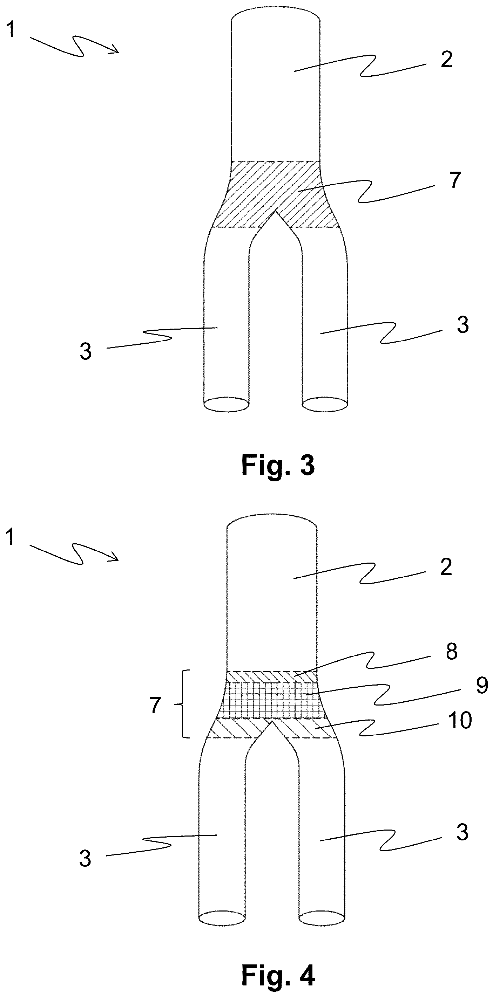

Bei der in Figur 3 gezeigten geflochtenen Struktur 1 ist ein Übergangsbereich 7 zwischen dem primären Schenkel 2 und den sekundären Schenkeln 3 vorgesehen. Dieser Übergangsbereich 7 ist aus dem gleichen Ensemble an Filamenten 4 geflochten, aus dem der primäre Schenkel 2 und die sekundären Schenkel 3 geflochten sind.

Wie auch bei den anderen Ausführungsbeispielen gezeigt, verlaufen die beiden sekundären Schenkel 3 im Wesentlichen parallel zu dem primären Schenkel 2. Die sekundären Schenkel 3 stehen somit nicht voneinander ab, sondern teilen den primären Schenkel 2 auf und verlaufen weiter im Wesentlichen in derselben Richtung wie der primäre Schenkel 2. Auch wenn es grundsätzlich möglich wäre, die sekundären Schenkel 3 von dem primären Schenkel 2 abstehen zu lassen, so ist es insbesondere im Sinne einer durchgängigen bzw. kontinuierlichen Fertigung der Struktur 1 unter gleichzeitiger Herstellung aller sekundären Schenkel 3 vorteilhaft, wenn die sekundären Schenkel 3 im Wesentlichen parallel zum primären Schenkel 2 verlaufen. Sollte für eine spezielle Anwendung das Abstehen der sekundären Schenkel 3 erforderlich sein, so kann dies beispielsweise mit einer Wärmenachbehandlung bewirkt werden.

Figur 4 zeigt eine mögliche Ausgestaltung des Übergangsbereichs 7. Dabei schließt an den primären Schenkel 2 ein Teilungsbereich 8 an. Dieser Teilungsbereich 8 überführt eine Flechtstruktur des primären Schenkels 2 in Flechtstrukturen der sekundären Schenkel 3. An den Teilungsbereich 8 schließt sodann ein Überkreuzungsbereich 9 an. Im Überkreuzungsbereich 9 überkreuzen sich Filamente 4, die dem ersten und dem zweiten sekundären Schenkel 3 zugeordnet sind. Dadurch wird das Loch 5, das ansonsten zwischen den sekundären Schenkeln 3 auftreten würde, geschlossen. An den Überkreuzungsbereich 9 schließt sich ein weiterer Teilungsbereich 10 an, der eine nach dem Überkreuzungsbereich 9 herrschende Flechtstruktur in die Flechtstrukturen der sekundären Schenkel 3 überführt. An diesen weiteren Teilungsbereich 10 schließen sodann die beiden sekundären Schenkel 3 an.

Selbstverständlich sind auch mehr als zwei sekundäre Schenkel 3 denkbar. Dann können im Überkreuzungsbereich 9 Überkreuzungen derart stattfinden, dass sich Filamente 4 von jedem der sekundären Schenkel 3 überkreuzen. Des Weiteren ist es möglich, dass nur ein Teilungsbereich 8 vorgesehen ist und/oder dass der Teilungsbereich 8 mit dem Überkreuzungsbereich 9 identisch ist, das heißt, dass im Rahmen des Überkreuzens der Filamente 4 auch gleich die Flechtstruktur vom primären Schenkel 2 in die Flechtstrukturen der sekundären Schenkel 3 übergeführt wird.

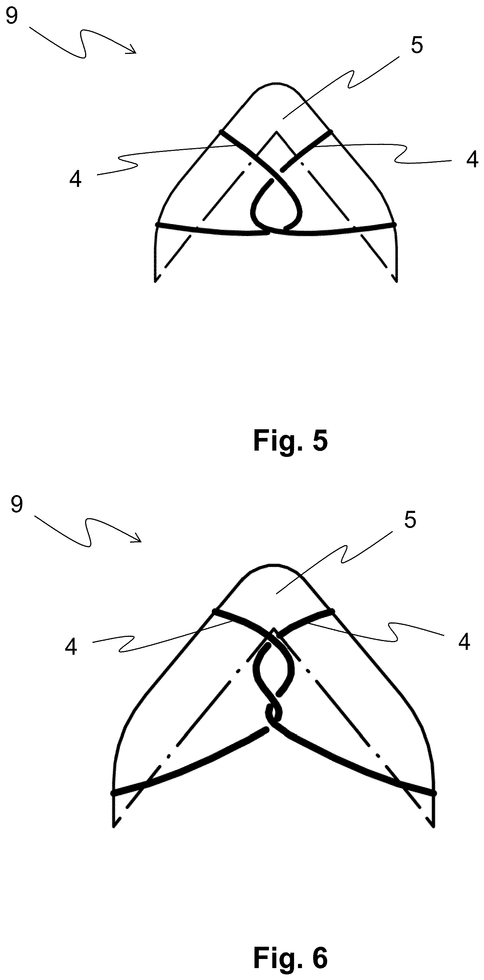

Figuren 5 bis 8 zeigen den Überkreuzungsbereich 9 am Übergang vom primären Schenkel 2 zu den sekundären Schenkeln 3, wo sich ohne die Überkreuzungen das Loch 5 befände.

Figur 5 zeigt eine einfache Überkreuzung von zwei Filamenten 4, die einer halben Umdrehung einer Verdrehung der Filamente 4 entspricht. Die Filamente 4 kehren dabei nach der Überkreuzung wieder zu den sekundären Schenkeln 3 zurück, von denen sie gekommen sind. Diese Überkreuzung führt zu einem einfachen Schließen des Lochs 5.

Figur 6 zeigt eine Verdrehung der Filamente 4 um eine ganze Umdrehung. Die Filamente 4 wechseln dabei vom einen sekundären Schenkel 3 zum anderen. Durch die längere Verdrehung der Filamente 4 wird das Loch 5 besser geschlossen als bei der einfachen Überkreuzung.

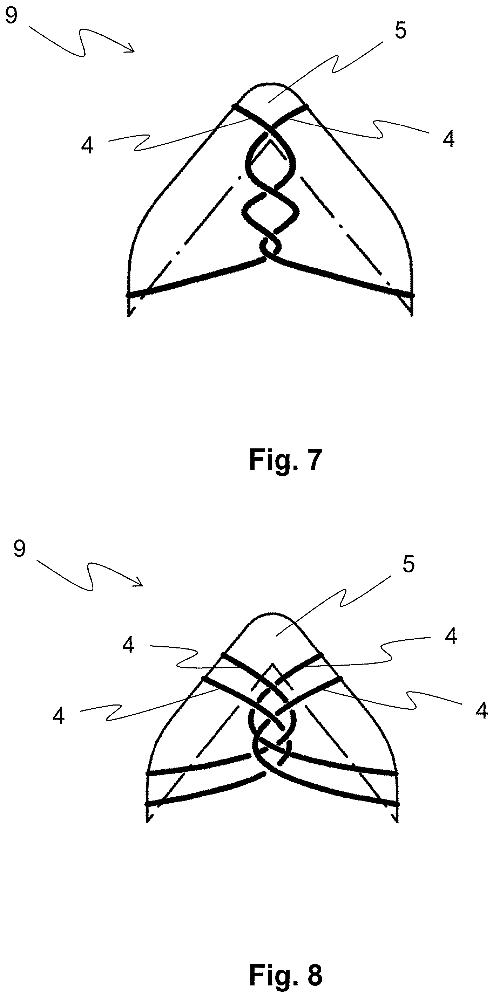

Eine Verdrehung der Filamente 4 um drei halbe Umdrehungen, wie in Figur 7 gezeigt, führt dazu, dass die Filamente 4 wieder zu den sekundären Schenkeln 3 zurückkehren, von denen sie gekommen sind. Das Loch 5 wird durch die relativ lange Verdrehung der Filamente 4 geschlossen.

Figur 8 zeigt ein weiteres Ausführungsbeispiel des Überkreuzungsbereichs 9, bei dem an der Überkreuzung jeweils zwei Filamente 4 der sekundären Schenkel 3 beteiligt sind. Die Überkreuzung ist dabei eine einfache Überkreuzung. Es ist aber auch möglich, mit zwei Filamenten 4 Verdrehungen um eine ganze Umdrehung oder mehr durchzuführen. Ebenso ist es möglich, dass sich mehr als zwei Filamente 4 pro sekundärem Schenkel 3 überkreuzen.

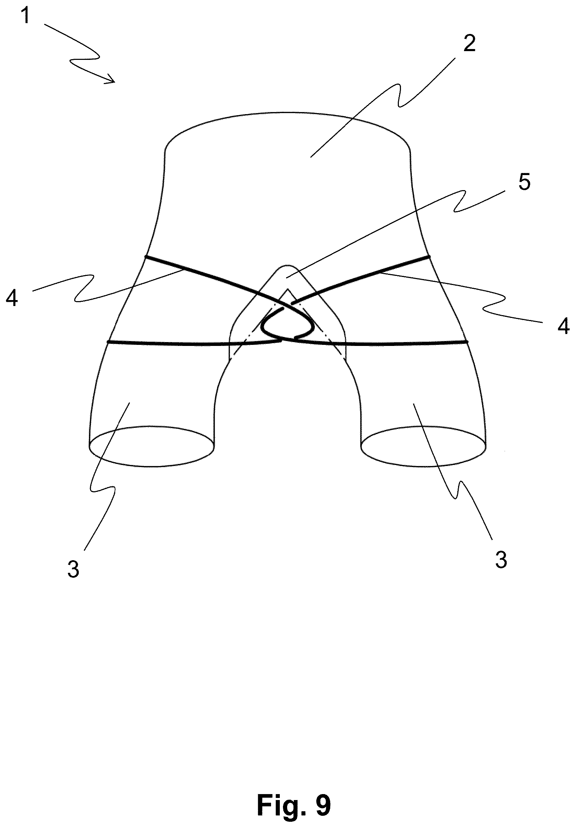

Bei dem in Figur 9 gezeigten Ausführungsbeispiel überkreuzen sich im Überkreuzungsbereich 9 Filamente 4, die von Seiten der sekundären Schenkel 3 kommen, die dem jeweils anderen sekundären Schenkel 3 abgewandt sind, mit anderen Worten überkreuzen sich Filamente 4 von den Außenseiten der sekundären Schenkel 3. Durch dieses Überkreuzen wird das Loch 5 geschlossen und die sekundären Schenkel 3 werden zusätzlich zusammengehalten.

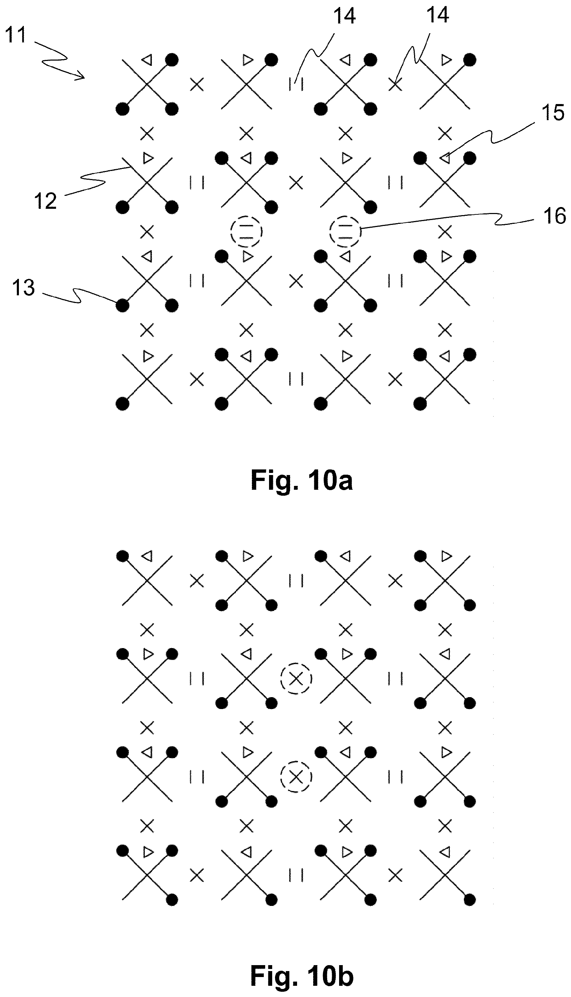

Figuren 10a bis 10c zeigen Flechtprogrammschritte für einen Teilungsbereich 8 auf einem Variationsflechter 11 mit 4 x 4 Flügelrädern 12. In Figur 10a ist dabei der Besatz mit Klöppeln 13 und die Stellung der Weichen 14 für das Flechten des primären Schenkels 2 dargestellt. Dabei bedeutet bei den Weichen 14 ein "×", dass ein Kreuzen der Klöppel 13 stattfindet, und ein "∥" bzw. "=", dass kein Kreuzen der Klöppel 13 stattfindet. Die Drehrichtung 15 der Flügelräder 12 ist durch ein kleines Dreieck angezeigt.

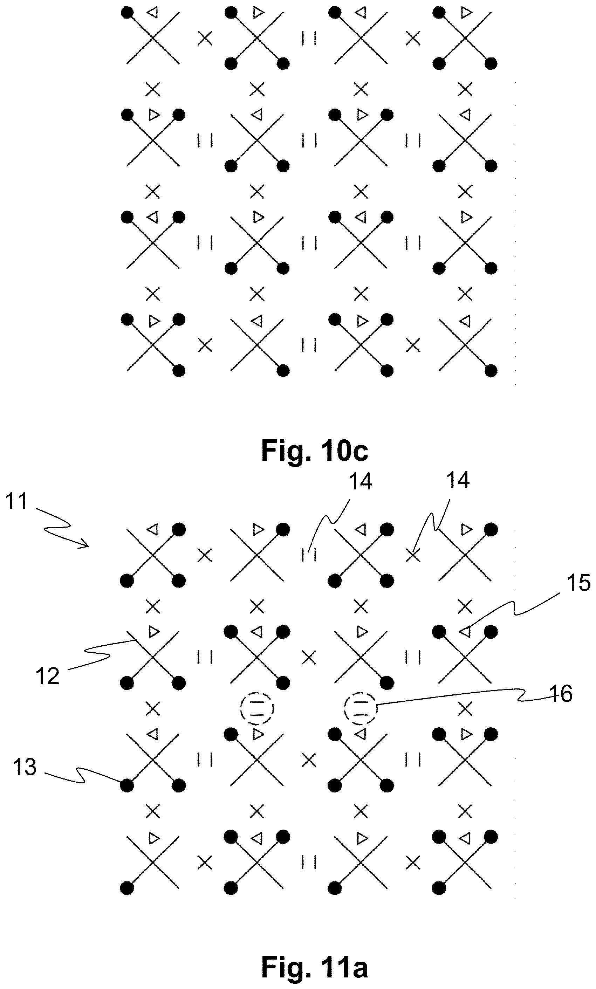

Für das Flechten des Teilungsbereiches 8 werden nun die umzustellenden Weichen 16, die durch eine gestrichelte Umrandung gekennzeichnet sind, umgestellt. Die Flügelräder 12 werden sodann um 90° weitergedreht. Es ergibt sich der Besatz mit Klöppeln 13 und die Stellung der Weichen 14 der Figur 10b. Zum Abschluss des Teilungsbereichs 8 werden die in Figur 10b umzustellenden Weichen 16 umgestellt. Es ergibt sich der Besatz mit Klöppeln 13 und die Stellung der Weichen 14 der Figur 10c, wonach zwei sekundäre Schenkel 3 geflochten werden.

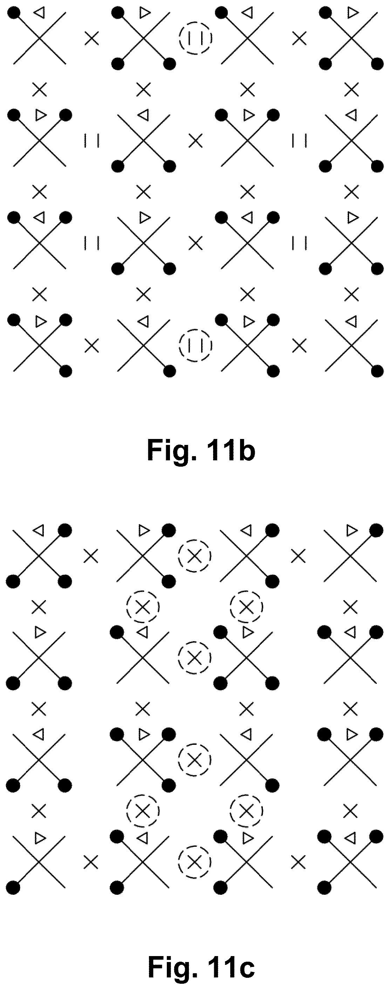

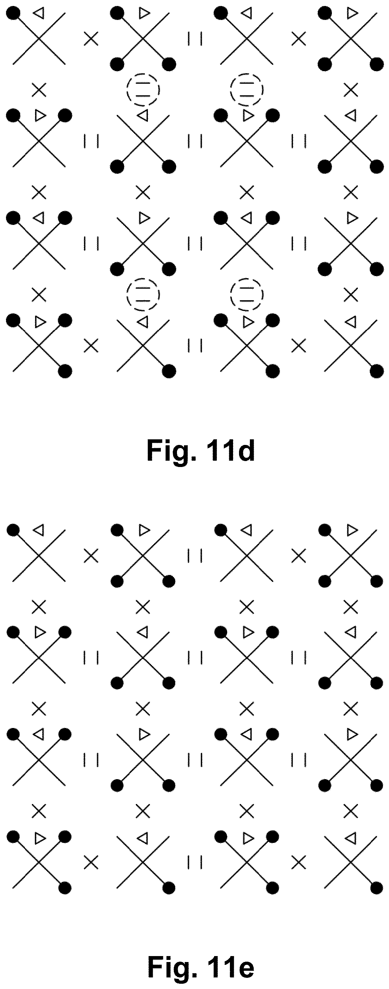

Figuren 11a bis 11e zeigen Flechtprogrammschritte für einen Übergangsbereich 7, der einen Teilungsbereich 8 und einen Überkreuzungsbereich 9 vereint. Der Ausgangspunkt der Figur 11a ist wie in Figur 10a der Besatz mit Klöppeln 13 und die Stellung der Weichen 14 für das Flechten des primären Schenkels 2. Nach Stellen der umzustellenden Weichen 16 und Drehung der Flügelräder 12 um 90° erhält man den Besatz mit Klöppeln 13 und die Stellung der Weichen 14 der Figur 11b. Es werden nun wieder die umzustellenden Weichen 16 gestellt und die Flügelräder 12 um 90° gedreht, um den Besatz mit Klöppeln 13 und die Stellung der Weichen der Figur 11c zu erhalten. Noch einmal werden die umzustellenden Weichen 16 gestellt und die Flügelräder 12 um 90° gedreht, um den Besatz mit Klöppeln 13 und die Stellung der Weichen der Figur 11d zu erhalten. Nach dem Stellen der umzustellenden Weichen 16 gelangt man zum Besatz mit Klöppeln 13 und zur Stellung der Weichen der Figur 11e, wonach wie in Figur 10c zwei sekundäre Schenkel geflochten werden. In vier kurzen Schritten werden also gleichzeitig der Teilungsbereich 8 und Überkreuzungsbereich 9 geflochten.

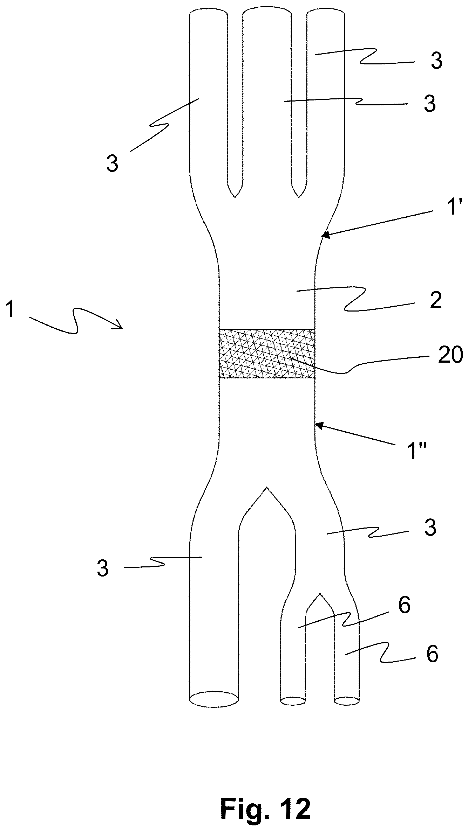

Figur 12 zeigt eine schematische Ansicht einer weiteren geflochtenen Struktur ähnlich Figur 2 mit einem Trennbereich 20 an dem primären Schenkel 2. Die hier gezeigte komplexe geflochtene Struktur 1 wird vorzugsweise in dem Trennbereich 20 auseinandergetrennt. Die Trennung kann beispielsweise durch einen Laserschnitt erfolgen. Hierdurch erhält man zwei geflochtene Strukturen 1' und 1". Der Trennbereich 20 kann alternativ oder zusätzlich an einem oder mehreren der sekundären Schenkel 3 bzw. 6 angeordnet sein (nicht gezeigt).

In dem Trennbereich 20 ist eine geänderte Maschenstruktur der Filamente 4 angedeutet. Beim Trennen der Struktur 1 in dem Trennbereich 20 kann hierdurch bewirkt werden, dass die Filamente 4 an dem dadurch entstehenden Ende der Struktur 1' bzw. 1" vorteilhaft, zum Beispiel kompakter, angeordnet sind und damit einen vorteilhaften Abschluss der Struktur 1' bzw. 1" bilden.

Selbstverständlich können mehrere Strukturen 1' bzw. 1" aneinandergereiht werden und dementsprechend auch mehrere Trennbereiche 20 vorgesehen sein (nicht gezeigt). Grundsätzlich ist eine Trennung einer solchen komplexen Struktur auch möglich, ohne dass Trennbereiche 20 vorgesehen sind. Die losen Enden der Filamente 4 können je nach Anwendungsfall entsprechend abstehen oder weiterverarbeitet werden, sodass beispielsweise durch Schlaufenbildung ein stumpfes Ende der Struktur 1' bzw. 1" erzeugt wird.

Die vorliegende Erfindung ist nicht auf die dargestellten und beschriebenen Ausführungsbeispiele beschränkt.

## Patentansprüche

1. Geflochtene Struktur, insbesondere Stent, mit einem primären Schenkel (2) und mindestens zwei sekundären Schenkeln (3), wobei der primäre Schenkel (2) aus einem Ensemble an Filamenten (4), insbesondere Fäden oder Drähten, geflochten ist und die sekundären Schenkel (3) aus Filamenten (4) geflochten sind, so dass die Gesamtheit der den sekundären Schenkeln (3) zugeordneten Filamente (4) gleich dem Ensemble an Filamenten (4) des primären Schenkels ist und
zwischen dem primären Schenkel (2) und den sekundären Schenkeln (3) ein aus dem Ensemble an Filamenten (4) des primären Schenkels (2) geflochtener Übergangsbereich (7) angeordnet ist, **dadurch gekennzeichnet,**
**dass** der geflochtene Übergangsbereich (7) im Wesentlichen frei von Löchern (5) ist,
**dass** der Übergangsbereich (7) aus dem gleichen Ensemble an Filamenten (4) geflochten ist, aus dem der primäre Schenkel (2) und die sekundären Schenkel (3) geflochten sind,
**dass** der Übergangsbereich (7) einen Überkreuzungsbereich (9) umfasst, in dem sich Filamente (4), die verschiedenen sekundären Schenkeln (3) zugeordnet sind, überkreuzen und
**dass** sich im Überkreuzungsbereich (9) auch Filamente (4) überkreuzen, die von Seiten des sekundären Schenkels (3) kommen, die dem jeweils anderen sekundären Schenkel (3) abgewandt sind.

2. Geflochtene Struktur nach dem vorherigen Anspruch, **dadurch gekennzeichnet, dass** die geflochtene Struktur (1) durchgängig, insbesondere auf einem Flechter mit verstellbaren Weichen, vorzugsweise einem Variationsflechter, Verzweigungsflechter oder 3D-Flechter (11), geflochten ist.

3. Geflochtene Struktur nach einem der vorherigen Ansprüche, **dadurch gekennzeichnet, dass** der Übergangsbereich (7) einen Teilungsbereich (8) umfasst, der eine Flechtstruktur des primären Schenkels (2) in Flechtstrukturen der sekundären Schenkel (3) überführt.

4. Geflochtene Struktur nach einem der vorherigen Ansprüche, **dadurch gekennzeichnet, dass** im Überkreuzungsbereich (9) zwei Filamente (4), die insbesondere verschiedenen sekundären Schenkeln (3) zugeordnet sind, untereinander verdreht sind.

5. Geflochtene Struktur nach einem der vorherigen Ansprüche, **dadurch gekennzeichnet, dass** der Übergangsbereich (7) einen weiteren Teilungsbereich (10) umfasst, so dass der Überkreuzungsbereich (9) zwischen dem Teilungsbereich (8) und dem weiteren Teilungsbereich (10) angeordnet ist.

6. Geflochtene Struktur nach einem der vorherigen Ansprüche, **dadurch gekennzeichnet, dass** an beiden Enden des primären Schenkels (2) sekundäre Schenkel (3) angeordnet sind.

7. Geflochtene Struktur nach einem der vorherigen Ansprüche, **dadurch gekennzeichnet, dass** an dem primären Schenkel (2) und/oder dem sekundären Schenkel (3) ein Trennbereich (20) mit einer gegenüber dem restlichen Schenkel (2,3) geänderter Maschendichte und/oder Maschenform zum Trennen der einen gesamten geflochtenen Struktur (1) in mehrere einzelne geflochtene Strukturen (1', 1") vorgesehen ist.

8. Verfahren zum Flechten einer geflochtenen Struktur (1), insbesondere eines Stents, mit einem primären Schenkel (2) und mindestens zwei sekundären Schenkeln (3), wobei aus einem Ensemble an Filamenten (4), insbesondere Fäden oder Drähten, zunächst der primäre Schenkel (2), sodann aus dem Ensemble an Filamenten (4) des primären Schenkels (2) ein im Wesentlichen lochfreier Übergangsbereich (7) und schließlich die sekundären Schenkel (3) geflochten werden, oder die Verfahrensschritte in umgekehrter Reihenfolge durchgeführt werden und die Gesamtheit der den sekundären Schenkeln (3) zugeordneten Filamente (4) gleich dem Ensemble an Filamenten (4) des primären Schenkels ist,
**dadurch gekennzeichnet,**
**dass** der geflochtene Übergangsbereich (7) im Wesentlichen frei von Löchern (5) ist,
**dass** der Übergangsbereich (7) aus dem gleichen Ensemble an Filamenten (4) geflochten ist, aus dem der primäre Schenkel (2) und die sekundären Schenkel (3) geflochten sind,
**dass** im Übergangsbereich (7) Filamente (4), die verschiedenen sekundären Schenkeln (3) zugeordnet sind, überkreuzt werden und somit einen Überkreuzungsbereich (9) ausbilden und
**dass** sich im Überkreuzungsbereich (9) auch Filamente (4) überkreuzen, die von Seiten des sekundären Schenkels (3) kommen, die dem jeweils anderen sekundären Schenkel (3) abgewandt sind.

9. Verfahren nach Anspruch 8, **dadurch gekennzeichnet, dass** die geflochtene Struktur (1) durchgängig, insbesondere mit einem Flechter mit schaltbaren Weichen, vorzugsweise einem Variationsflechter, Verzweigungsflechter oder 3D-Flechter (11), geflochten wird.

10. Verfahren nach einem der Ansprüche 8 oder 9, **dadurch gekennzeichnet, dass** im Übergangsbereich (7) die Filamente (4) so geflochten werden, dass sie von einer Flechtstruktur des primären Schenkels (2) in Flechtstrukturen der sekundären Schenkel (3) überführt werden und somit einen Teilungsbereich (8) ausbilden.

11. Verfahren nach Anspruch 10, **dadurch gekennzeichnet, dass** im Überkreuzungsbereich (9) zwei Filamente (4), die insbesondere verschiedenen sekundären Schenkeln (3) zugeordnet sind, untereinander verdreht werden.

12. Verfahren nach einem der Ansprüche 10 oder 11, **dadurch gekennzeichnet, dass** im Überkreuzungsbereich (9) auch Filamente (4) miteinander verdreht werden, die von Seiten des sekundären Schenkels (3) kommen, die dem jeweils anderen sekundären Schenkel (3) abgewandt sind.

13. Verfahren nach einem der vorherigen Ansprüche 10 bis 12, **dadurch gekennzeichnet, dass** im Übergangsbereich (7) ein weiterer Teilungsbereich (10) geflochten wird, so dass zunächst der Teilungsbereich (8), dann der Überkreuzungsbereich (9) und schließlich der weitere Teilungsbereich (10) geflochten werden.

14. Verfahren nach einem der Ansprüche 8 bis 13, **dadurch gekennzeichnet, dass** die geflochtene Struktur (1), insbesondere in einem Trennbereich (20), in mehrere geflochtene Strukturen (1',1") getrennt wird.

## Claims

1. Braided structure, in particular stent, having a primary limb (2) and at least two secondary limbs (3), wherein the primary limb (2) is braided from an ensemble of filaments (4), in particular threads or wires, and the secondary limbs (3) are braided from filaments (4), such that the totality of the filaments (4) assigned to the secondary limbs (3) is equal to the ensemble of filaments (4) of the primary limb, and
a transition region (7) braided from the ensemble of filaments (4) of the primary limb (2) is arranged between the primary limb (2) and the secondary limbs (3), **characterized in that**
the braided transition region (7) is substantially free of holes (5),
the transition region (7) is braided from the same ensemble of filaments (4) from which the primary limb (2) and the secondary limbs (3) are braided,
the transition region (7) comprises a crossing region (9) in which filaments (4) which are assigned to different secondary limbs (3) cross, and
filaments (4) which come from sides of the secondary limb (3) which face away from the respective other secondary limb (3) also cross in the crossing region (9).

2. Braided structure according to the preceding claim, **characterized in** that the braided structure (1) is braided continuously, in particular on a braider with adjustable switches, preferably a variation braider, branching braider or 3D braider (11).

3. Braided structure according to one of the preceding claims, **characterized in that** the transition region (7) comprises a division region (8) which converts a braided structure of the primary limb (2) into braided structures of the secondary limbs (3).

4. Braided structure according to one of the preceding claims, **characterized in that** two filaments (4) which are assigned in particular to different secondary limbs (3) are twisted with respect to one another in the crossing region (9).

5. Braided structure according to one of the preceding claims, **characterized in that** the transition region (7) comprises a further division region (10), with the result that the crossing region (9) is arranged between the division region (8) and the further division region (10).

6. Braided structure according to one of the preceding claims, **characterized in that** secondary limbs (3) are arranged at both ends of the primary limb (2).

7. Braided structure according to one of the preceding claims, **characterized in that** a separation region (20) with a mesh density and/or mesh shape which is changed in relation to the remaining limb (2, 3) for separating the one entire braided structure (1) into a plurality of individual braided structures (1', 1") is provided on the primary limb (2) and/or the secondary limb (3).

8. Method for braiding a braided structure (1), in particular a stent, having a primary limb (2) and at least two secondary limbs (3), wherein firstly the primary limb (2) is braided from an ensemble of filaments (4), in particular threads or wires, then a substantially hole-free transition region (7) and finally the secondary limbs (3) are braided from the ensemble of filaments (4) of the primary limb (2), or the method steps are carried out in the reverse order and the totality of the filaments (4) assigned to the secondary limbs (3) is equal to the ensemble of filaments (4) of the primary limb,
**characterized in that**
the braided transition region (7) is substantially free of holes (5),
the transition region (7) is braided from the same ensemble of filaments (4) from which the primary limb (2) and the secondary limbs (3) are braided,
filaments (4) which are assigned to different secondary limbs (3) are crossed in the transition region (7) and thus form a crossing region (9), and
filaments (4) which come from sides of the secondary limb (3) which face away from the respective other secondary limb (3) also cross in the crossing region (9).

9. Method according to claim 8, **characterized in that** the braided structure (1) is braided continuously, in particular with a braider with switchable switches, preferably a variation braider, branching braider or 3D braider (11).

10. Method according to one of claims 8 or 9, **characterized in that** the filaments (4) are braided in the transition region (7) such that they are converted from a braided structure of the primary limb (2) into braided structures of the secondary limbs (3) and thus form a division region (8).

11. Method according to claim 10, **characterized in that** two filaments (4) which are assigned in particular to different secondary limbs (3) are twisted with respect to one another in the crossing region (9).

12. Method according to one of claims 10 or 11, **characterized in that** filaments (4) which come from sides of the secondary limb (3) which face away from the respective other secondary limb (3) also are twisted with respect to one another in the crossing region (9).

13. Method according to one of the preceding claims 10 to 12, **characterized in that** a further division region (10) is braided in the transition region (7), with the result that firstly the division region (8), then the crossing region (9) and finally the further division region (10) are braided.

14. Method according to one of claims 8 to 13, **characterized in that** the braided structure (1) is separated, in particular in a separation region (20), into a plurality of braided structures (1', 1").

## Revendications

1. Structure tressée, en particulier stent, comprenant une branche primaire (2) et au moins deux branches secondaires (3), la branche primaire (2) étant tressée à partir d'un ensemble de filaments (4), en particulier de fils ou de câbles, et les branches secondaires (3) étant tressées à partir de filaments (4) de telle sorte que l'ensemble des filaments (4) associés aux branches secondaires (3) soit identique à l'ensemble de filaments (4) de la branche primaire, et
une région de transition (7) tressée à partir de l'ensemble de filaments (4) de la branche primaire (2) étant disposée entre la branche primaire (2) et les branches secondaires (3), **caractérisée en ce que**
la région de transition tressée (7) est sensiblement exempte de trous (5),
la région de transition (7) est tressée à partir du même ensemble de filaments (4) à partir duquel la branche primaire (2) et les branches secondaires (3) sont tressées,
la région de transition (7) comprend une région de croisement (9) dans laquelle des filaments (4) qui sont associés à différentes branches secondaires (3) se croisent, et
dans la région de croisement (9), des filaments (4) qui viennent de côtés de la branche secondaire (3) qui sont opposés à l'autre branche secondaire respective (3) se croisent également.

2. Structure tressée selon la revendication précédente, **caractérisée en ce que** la structure tressée (1) est tressée de manière continue, en particulier sur un tresseur avec des aiguillages réglables, de préférence un tresseur à variation, un tresseur à ramification ou un tresseur 3D (11).

3. Structure tressée selon l'une quelconque des revendications précédentes, **caractérisée en ce que** la région de transition (7) comprend une région de division (8) qui transforme une structure tressée de la branche primaire (2) en structures tressées des branches secondaires (3).

4. Structure tressée selon l'une quelconque des revendications précédentes, **caractérisée en ce que** dans la région de croisement (9), deux filaments (4), qui sont en particulier associés à différentes branches secondaires (3), sont torsadés l'un par rapport à l'autre.

5. Structure tressée selon l'une quelconque des revendications précédentes, **caractérisée en ce que** la région de transition (7) comprend une région de division supplémentaire (10) de sorte que la région de croisement (9) est disposée entre la région de division (8) et la région de division supplémentaire (10).

6. Structure tressée selon l'une quelconque des revendications précédentes, **caractérisée en ce que** des branches secondaires (3) sont disposées aux deux extrémités de la branche primaire (2).

7. Structure tressée selon l'une quelconque des revendications précédentes, **caractérisée en ce qu'**une région de séparation (20) avec une densité de mailles et/ou une forme de mailles modifiée par rapport au reste de la branche (2, 3) est prévue au niveau de la branche primaire (2) et/ou de la branche secondaire (3) pour séparer l'ensemble de la structure tressée (1) en plusieurs structures tressées individuelles (1', 1").

8. Procédé pour tresser une structure tressée (1), en particulier un stent, comprenant une branche primaire (2) et au moins deux branches secondaires (3), la branche primaire (2) étant tout d'abord tressée à partir d'un ensemble de filaments (4), en particulier de fils ou de câbles, puis une région de transition (7) sensiblement exempte de trous étant tressée à partir de l'ensemble de filaments (4) de la branche primaire (2) et finalement les branches secondaires (3), ou les étapes du procédé étant réalisées dans l'ordre inverse et l'ensemble des filaments (4) associés aux branches secondaires (3) étant identique à l'ensemble de filaments (4) de la branche primaire,
**caractérisé en ce que**
la région de transition tressée (7) est sensiblement exempte de trous (5),
la région de transition (7) est tressée à partir du même ensemble de filaments (4) à partir duquel la branche primaire (2) et les branches secondaires (3) sont tressées,
dans la région de transition (7), des filaments (4) qui sont associés à différentes branches secondaires (3) se croisent et forment ainsi une région de croisement (9), et
dans la région de croisement (9), des filaments (4) qui viennent de côtés de la branche secondaire (3) qui sont opposés à l'autre branche secondaire respective (3) se croisent également.

9. Procédé selon la revendication 8, **caractérisé en ce que** la structure tressée (1) est tressée de manière continue, en particulier avec un tresseur avec des aiguillages commutables, de préférence un tresseur à variation, un tresseur à ramification ou un tresseur 3D (11).

10. Procédé selon l'une quelconque des revendications 8 ou 9, **caractérisé en ce que** dans la région de transition (7), les filaments (4) sont tressés de sorte qu'ils sont transformés d'une structure tressée de la branche primaire (2) en structures tressées des branches secondaires (3) et forment ainsi une région de division (8).

11. Procédé selon la revendication 10, **caractérisé en ce que** dans la région de croisement (9), deux filaments (4), qui sont en particulier associés à différentes branches secondaires (3), sont torsadés l'un par rapport à l'autre.

12. Procédé selon l'une quelconque des revendications 10 ou 11, **caractérisé en ce que** dans la région de croisement (9), des filaments (4) qui viennent de côtés de la branche secondaire (3) qui sont opposés à l'autre branche secondaire respective (3) sont également torsadés l'un par rapport à l'autre.

13. Procédé selon l'une quelconque des revendications précédentes 10 à 12, **caractérisé en ce que** dans la région de transition (7), une région de division supplémentaire (10) est tressée de sorte que tout d'abord la région de division (8), puis la région de croisement (9) et finalement la région de division supplémentaire (10) sont tressées.

14. Procédé selon l'une quelconque des revendications 8 à 13, **caractérisé en ce que** la structure tressée (1), en particulier dans une région de séparation (20), est séparée en plusieurs structures tressées (1', 1").
